# EUROPEAN PATENT APPLICATION

(11) **EP 2 668 933 A1**
(43) Date of publication of application: **04.12.2013**
(21) Application number: 13003006.7
(22) Date of filing: 22.09.2003
(51) Int. Cl.: A61F 2/915, A61L 31/16

(54) **Expandable medical device with openings for delivery of multiple beneficial agents**

(30) Priority: 20.09.2002 US 412489 P
(62) Divisional of application: 03797926.7
(71) Applicant: Innovational Holdings, LLC, New Brunswick, NJ 08933 (US)
(72) Inventor: Shanley, John F., Emerald Hills, CA 94062-4072 (US)
(74) Representative: Diehl & Partner GbR

(57) **Abstract**

A tissue supporting device is disclosed, comprising: a generally cylindrical tissue supporting device body (300;400;500;600) configured to support a bodily lumen; a plurality of first through openings (330a;430a; 530a;630a) in the tissue supporting device body (300; 400;500;600), wherein the first openings (330a;430a; 530a;630a) contain a first beneficial agent; and a plurality of second through openings (330b;430b;530b; 630b) in the tissue supporting device body (300;400; 500;600), wherein the second openings (330b;430b;530b; 630b) contain a second beneficial agent, characterized in that the second beneficial agent is different from the first beneficial agent.

## Description

### CROSS REFERENCE TO RELATED APPLICATIONS

This application claims priority under 35 U.S.C. §119(e) to U.S. Provisional Application Serial No. 60/412,489 filed September 20, 2002, which is incorporated herein by reference in its entirety.

### BACKGROUND OF THE INVENTION

### Field of the Invention

The present invention relates to tissue-supporting medical devices, and more particularly to expandable, non-removable devices that are implanted within a bodily lumen of a living animal or human to support the organ and maintain patency, and that have openings for delivery of a plurality of beneficial agents to the intervention site.

### Summary of the Related Art

In the past, permanent or biodegradable devices have been developed for implantation within a body passageway to maintain patency of the passageway. These devices are typically introduced percutaneously, and transported transluminally until positioned at a desired location. These devices are then expanded either mechanically, such as by the expansion of a mandrel or balloon positioned inside the device, or expand themselves by releasing stored energy upon actuation within the body. Once expanded within the lumen, these devices, called stents, become encapsulated within the body tissue and remain a permanent implant.

Known stent designs include monofilament wire coil stents (U.S. Pat. No. 4,969,458); welded metal cages (U.S. Pat. Nos. 4,733,665 and 4,776,337); and, most prominently, thin-walled metal cylinders with axial slots formed around the circumference (U.S. Pat. Nos. 4,733,665; 4,739,762; and 4,776,337). Known construction materials for use in stents include polymers, organic fabrics and biocompatible metals, such as, stainless steel, gold, silver, tantalum, titanium, and shape memory alloys, such as Nitinol.

U.S. Pat. Nos. 4,733,665; 4,739,762; and 4,776,337 disclose expandable and deformable interluminal vascular grafts in the form of thin-walled tubular members with axial slots allowing the members to be expanded radially outwardly into contact with a body passageway. After insertion, the tubular members are mechanically expanded beyond their elastic limit and thus permanently fixed within the body. U.S. Pat. No. 5,545,210 discloses a thin-walled tubular stent geometrically similar to those discussed above, but constructed of a nickel-titanium shape memory alloy ("Nitinol"), which can be permanently fixed within the body without exceeding its elastic limit. All of these stents share a critical design property: in each design, the features that undergo permanent deformation during stent expansion are prismatic, i.e., the cross sections of these features remain constant or change very gradually along their entire active length. These prismatic structures are ideally suited to providing large amounts of elastic deformation before permanent deformation commences, which in turn leads to sub-optimal device performance in important properties including stent expansion force, stent recoil, strut element stability, stent securement on delivery catheters and radiopacity.

U.S. Pat. No. 6,241,762 which is incorporated herein by reference in its entirety, discloses a non-prismatic stent design which remedies the above mentioned performance deficiencies of previous stents. In addition, preferred embodiments of this patent provide a stent with large, non-deforming strut and link elements, which can contain holes without compromising the mechanical properties of the strut or link elements, or the device as a whole. Further, these holes may serve as large, protected reservoirs for delivering various beneficial agents to the device implantation site.

Of the many problems that may be addressed through stent-based local delivery of beneficial agents, one of the most important is restenosis. Restenosis is a major complication that can arise following vascular interventions such as angioplasty and the implantation of stents. Simply defined, restenosis is a wound healing process that reduces the vessel lumen diameter by extracellular matrix deposition and vascular smooth muscle cell proliferation and which may ultimately result in renarrowing or even reocclusion of the lumen. Despite the introduction of improved surgical techniques, devices and pharmaceutical agents, the overall restenosis rate is still reported in the range of 25% to 50% within six to twelve months after an angioplasty procedure. To treat this condition, additional revascularization procedures are frequently required, thereby increasing trauma and risk to the patient.

Conventional stents with surface coatings of various beneficial agents have shown promising early results. U.S. Pat. No. 5,716,981, for example, discloses a stent that is surface-coated with a composition comprising a polymer carrier and paclitaxel (a well-known compound that is commonly used in the treatment of cancerous tumors). The patent offers detailed descriptions of methods for coating stent surfaces, such as spraying and dipping, as well as the desired character of the coating itself: it should "coat the stent smoothly and evenly" and "provide a uniform, predictable, prolonged release of the anti-angiogenic factor." Surface coatings, however, can provide little actual control over the release kinetics of beneficial agents. These coatings are necessarily very thin, typically 5 to 8 microns deep. The surface area of the stent, by comparison is very large, so that the entire volume of the beneficial agent has a very short diffusion path to discharge into the surrounding tissue. The resulting cumulative drug release profile is characterized by a large initial burst, followed by a rapid approach to an asymptote, rather than the desired "uniform, prolonged release," or linear release.

Increasing the thickness of the surface coating has the beneficial effects of improving drug release kinetics including the ability to control drug release and to allow increased drug loading. However, the increased coating thickness results in increased overall thickness of the stent wall. This is undesirable for a number of reasons, including increased trauma to the vessel lumen during implantation, reduced flow cross-section of the lumen after implantation, and increased vulnerability of the coating to mechanical failure or damage during expansion and implantation. Coating thickness is one of several factors that affect the release kinetics of the beneficial agent, and limitations on thickness thereby limit the range of release rates, durations, and the like that can be achieved.

Recent research described in a paper titled "Physiological Transport Forces Govern Drug Distribution for Stent-Based Delivery" by Chao-Wei Hwang et al. has revealed an important interrelationship between the spatial and temporal drug distribution properties of drug eluting stents, and cellular drug transport mechanisms. In pursuit of enhanced mechanical performance and structural properties stent designs have evolved to more complex geometries with inherent inhomogeneity in the circumferential and longitudinal distribution of stent struts. Examples of this trend are the typical commercially available stents which expand to a roughly diamond or hexagonal shape when deployed in a bodily lumen. Both have been used to deliver a beneficial agent in the form of a surface coating. Studies have shown that lumen tissue portions immediately adjacent to the struts acquire much higher concentrations of drug than more remote tissue portions, such as those located in the middle of the "diamond" shaped strut cells. Significantly, this concentration gradient of drug within the lumen wall remains higher over time for hydrophobic beneficial agents, such as paclitaxel or Rapamycin, which have proven to be the most effective anti-proliferatives to date. Because local drug concentrations and gradients are inextricably linked to biological effect, the initial spatial distribution of the beneficial agent sources (the stent struts) is key to efficacy.

In addition to sub-optimal spatial distribution of beneficial agents, there are further problems with surface coated stents. The fixed matrix polymer carriers frequently used in the device coatings typically retain approximately 30% of the beneficial agent in the coating indefinitely. Since these beneficial agents are frequently highly cytotoxic, sub-acute and chronic problems such as chronic inflammation, late thrombosis, and late or incomplete healing of the vessel wall may occur. Additionally, the carrier polymers themselves are often inflammatory to the tissue of the vessel wall. On the other hand, use of bio-degradable polymer carriers on stent surfaces can result in "mal-apposition" or voids between the stent and tissue of the vessel wall after the polymer carrier has degraded. The voids permit differential motion between the stent and adjacent tissue. Resulting problems include micro-abrasion and inflammation, stent drift, and failure to re-endothelialize the vessel wall.

Early human clinical trials suggest that there may be further problems with first generation drug delivery devices. Follow-up examination of clinical trial patients at 6 to 18 months after drug coated stent implantation indicates that mal-apposition of stent struts to arterial walls and edge effect restenosis may occur in significant numbers of patients. Edge effect restenosis occurs just beyond the proximal and distal edges of the stent and progresses around the stent edges and into the interior (luminal) space, frequently requiring repeat revascularization of the patient.

Another significant problem is that expansion of the stent may stress an overlying polymeric coating causing the coating to peel, crack, or rupture which may effect drug release kinetics or have other untoward effects. These effects have been observed in first generation drug coated stents when these stents are expanded to larger diameters, preventing their use thus far in larger diameter arteries. Further, expansion of such a coated stent in an atherosclerotic blood vessel will place circumferential shear forces on the polymeric coating, which may cause the coating to separate from the underlying stent surface. Such separation may again have untoward effects including embolization of coating fragments causing vascular obstruction.

### SUMMARY OF THE INVENTION

In view of the drawbacks of the prior art, it would be advantageous to provide a stent capable of delivering a relatively large volume of a beneficial agent to a traumatized site in a vessel lumen while avoiding the numerous problems associated with surface coatings containing beneficial agents, without increasing the effective wall thickness of the stent, and without adversely impacting the mechanical expansion properties of the stent.

It would further be advantageous to have a tissue supporting device which improves the spatial distribution of beneficial agents in lumen tissue by allowing variations in doses or concentrations of beneficial agents within the device.

It would further be advantageous to provide a tissue supporting device with different beneficial agents provided in different holes to achieve a desired spatial distribution of two or more beneficial agents.

It would further be advantageous to provide a tissue supporting device with different beneficial agents provided at the edges of the device and within boundaries of the device, in order to prevent undesirable edge effects.

It would also be advantageous to provide a tissue supporting device in which a beneficial agent is provided within the boundaries of the device, and the beneficial agent is provided in higher or lower concentrations or excluded altogether from the edges of the device, in order to prevent undesirable edge effects.

In accordance with one aspect of the present invention, an expandable medical device for delivery of a beneficial agent includes a substantially cylindrical device which is expandable from a cylinder having a first diameter to a cylinder having a second diameter, a first plurality of openings formed in the substantially cylindrical device containing a first beneficial agent for delivery to tissue, and a second plurality of openings formed in the substantially cylindrical device containing a second beneficial agent for delivery to tissue. The first openings are positioned on first and second ends of the cylindrical device. The second openings are positioned on a central portion of the cylindrical device between the first and second ends and the second beneficial agent is different than the first beneficial agent.

In accordance with an additional aspect of the present invention, a tissue supporting device includes a tissue supporting device body configured to support a bodily lumen, a first beneficial agent contained in first openings in the tissue supporting device for delivery to tissue, and a second beneficial agent contained in second openings in the tissue supporting device for delivery to tissue. The first openings are positioned on first and second ends of the device body. The second openings are positioned on a central portion of the device body between the first and second ends.

In accordance with a further aspect of the present invention, an expandable medical device for delivery of a beneficial agent includes a device body which is expandable from an initial configuration to an expanded configuration, a side opening in the device body configured to accommodate a bifurcation in a lumen, and a first plurality of openings formed in the device body containing a first beneficial agent for delivery to tissue at the expanded configuration. The first openings are formed in an area surrounding the side opening. A second plurality of openings are formed in the body device in an area away from the side opening.

In accordance with another aspect of the present invention, a method of reducing restenosis in a body passageway includes the steps of positioning a tissue supporting device in a body passageway to support the tissue, the tissue supporting device containing a first and a second beneficial agent in openings in the device, and delivering the first beneficial agent to tissue at locations adjacent ends of the tissue supporting device and the second beneficial agent to tissue between the ends of the device to reduce restenosis.

In accordance with a further aspect of the present invention, an expandable medical device for delivery of a beneficial agent includes a substantially cylindrical device which is expandable from a cylinder having a first diameter to a cylinder having a second diameter, a first plurality of openings formed in the substantially cylindrical device containing a beneficial agent in a first concentration, and a second plurality of openings formed in the substantially cylindrical device containing the beneficial agent in a second concentration. The first and second openings are arranged to deliver a uniform distribution of a drug to the tissue of a body passageway.

### BRIEF DESCRIPTION OF THE DRAWINGS

The invention will now be described in greater detail with reference to the preferred embodiments illustrated in the accompanying drawings, in which like elements bear like reference numerals, and wherein:

FIG. 1 is an isometric view of an expandable medical device with a beneficial agent at the ends;

FIG. 2 is an isometric view of an expandable medical device with a beneficial agent at a central portion and no beneficial agent at the ends;

FIG. 3 is an isometric view of an expandable medical device with different beneficial agents in different holes;

FIG. 4 is an isometric view of an expandable medical device with different beneficial agents in alternating holes;

FIG. 5 is an enlarged side view of a portion of an expandable medical device with beneficial agent openings in the bridging elements; and

FIG. 6 is an enlarged side view of a portion of an expandable medical device with a bifurcation opening.

### DETAILED DESCRIPTION OF THE PREFERRED EMBODIMENTS

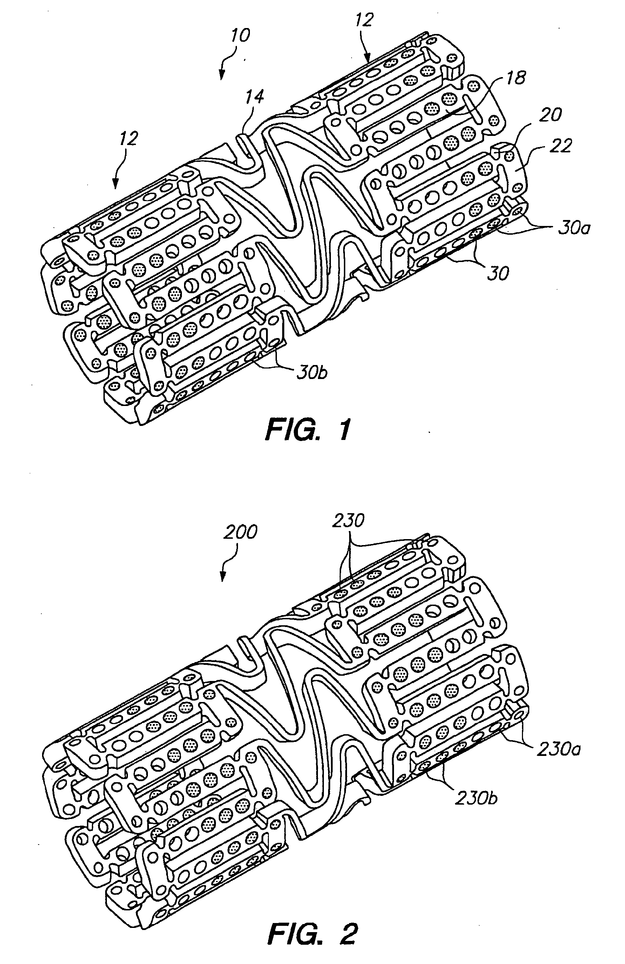

FIG. 1 illustrates an expandable medical device having a plurality of holes containing a beneficial agent for delivery to tissue by the expandable medical device. The expandable medical device 10 shown in FIG. 1 is cut from a tube of material to form a cylindrical expandable device. The expandable medical device 10 includes a plurality of cylindrical sections 12 interconnected by a plurality of bridging elements 14. The bridging elements 14 allow the tissue supporting device to bend axially when passing through the torturous path of vasculature to a deployment site and allow the device to bend axially when necessary to match the curvature of a lumen to be supported. Each of the cylindrical tubes 12 is formed by a network of elongated struts 18 which are interconnected by ductile hinges 20 and circumferential struts 22. During expansion of the medical device 10 the ductile hinges 20 deform while the struts 18 are not deformed. Further details of one example of the expandable medical device are described in U.S. Patent No. 6,241,762 which is incorporated herein by reference in its entirety.

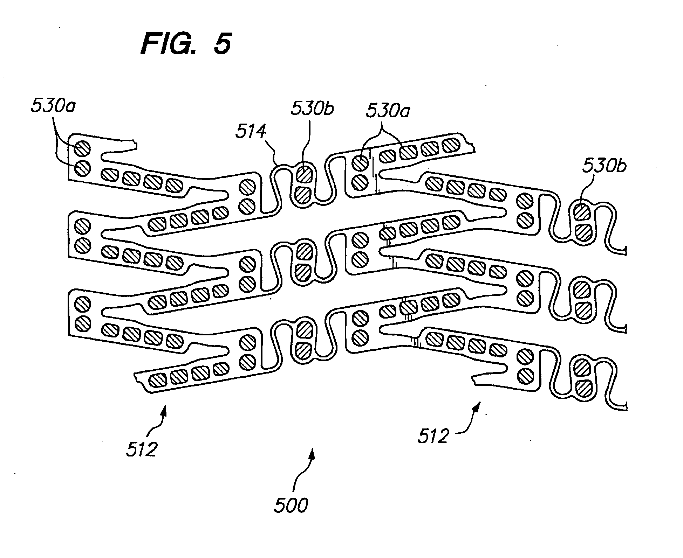

As shown in FIG. 1, the elongated struts 18 and circumferential struts 22 include openings 30, some of which contain a beneficial agent for delivery to the lumen in which the expandable medical device is implanted. In addition, other portions of the device 10, such as the bridging elements 14, may include openings, as discussed below with respect to FIG. 5. Preferably, the openings 30 are provided in non-deforming portions of the device 10, such as the struts 18, so that the openings are non-deforming and the beneficial agent is delivered without risk of being fractured, expelled, or otherwise damaged during expansion of the device. A further description of one example of the manner in which the beneficial agent may be loaded within the openings 30 is described in U.S. Patent Application Serial No. 09/948,987, filed September 7, 2001, which is incorporated herein by reference in its entirety.

The embodiments of the invention shown can be further refined by using Finite Element Analysis and other techniques to optimize the deployment of the beneficial agents within the openings 30. Basically, the shape and location of the openings 30, can be modified to maximize the volume of the voids while preserving the relatively high strength and rigidity of the struts with respect to the ductile hinges 20. According to one preferred embodiment of the present invention, the openings have an area of at least 5 x 10⁻⁶ square inches, and preferably at least 7 x 10⁻⁶ square inches. Typcially, the openings are filled about 50% to about 95% full of beneficial agent.

### Definitions

The terms "agent" or "beneficial agent" as used herein are intended to have the broadest possible interpretation and are used to include any therapeutic agent or drug, as well as inactive agents such as barrier layers, carrier layers, therapeutic layers, or protective layers.

The terms "drug" and "therapeutic agent" are used interchangeably to refer to any therapeutically active substance that is delivered to a bodily lumen of a living being to produce a desired, usually beneficial, effect. Beneficial agents may include one or more drug or therapeutic agent.

The present invention is particularly well suited for the delivery of antineoplastics, antiangiogenics, angiogenic factors, anti-inflammatories, immunosuppressants, antirestenotics, antiplatelet agents, vasodilators, anti-thrombotics, antiproliferatives, such as paclitaxel and Rapamycin, for example, and antithrombins, such as heparin, for example.

The therapeutic agents for use with the present invention also include classical low molecular weight therapeutic agents (commonly referred to as drugs) including but not limited to immunosuppressants, antilipid agents, anti-inflammatory agents, vitamins, antimitotics, metalloproteinase inhibitors, NO donors, estradiols, anti-sclerosing agents, and vasoactive agents, endothelial growth factors, estrogen, beta blockers, AZ blockers, hormones, statins, insulin growth factors, antioxidants, membrane stabilizing agents, calcium antagonists, retenoid, alone or in combinations with any therapeutic agent mentioned herein. Therapeutic agents also include peptides, lipoproteins, polypeptides, polynucleotides encoding polypeptides, lipids, protein-drugs, enzymes, oligonucleotides and their derivatives, ribozymes, other genetic material, cells antisense, monoclonal antibodies, platelets, prions, viruses, bacteria, and eukaryotic cells such as endothelial cells, ACE inhibitors, monocyte/macrophages or vascular smooth muscle cells to name but a few examples. The therapeutic agent may also be a pro-drug, which metabolizes into the desired drug when administered to a host. In addition, therapeutic agents may be pre-formulated as microcapsules, microspheres, microbubbles, liposomes, niosomes, emulsions, dispersions or the like before they are incorporated into the therapeutic layer. Therapeutic agents may also be radioactive isotopes or agents activated by some other form of energy such as light or ultrasonic energy, or by other circulating molecules that can be systemically administered. Therapeutic agents may perform multiple functions including modulating angiogenesis, restenosis, cell proliferation, thrombosis, platelet aggregation, clotting and vasodilation. Anti-inflammatories include non-steroidal anti-inflammatories (NSAID), such as aryl acetic acid derivatives, e.g., Diclofenac; aryl propionic acid derivatives, e.g., Naproxen; and salicylic acid derivatives, e.g., aspirin, Diflunisal. Anti-inflammatories also include glucocoriticoids (steroids) such as dexamethasone, prednisolone, and triamcinolone. Anti-inflammatories may be used in combination with antiproliferatives to mitigate the reaction of the tissue to the antiproliferative.

The term "erosion" means the process by which components of a medium or matrix are bioresorbed and/or degraded and/or broken down by chemical or physical processes. For example in reference to biodegradable polymer matrices, erosion can occur by cleavage or hydrolysis of the polymer chains, thereby increasing the solubility of the matrix and suspended beneficial agents.

The term "erosion rate" is a measure of the amount of time it takes for the erosion process to occur, usually reported in unit-area per unit-time.

The terms "matrix" or "bioresorbable matrix" are used interchangeably to refer to a medium or material that, upon implantation in a subject, does not elicit a detrimental response sufficient to result in the rejection of the matrix. The matrix typically does not provide any therapeutic responses itself, though the matrix may contain or surround a beneficial agent, as defined herein. A matrix is also a medium that may simply provide support, structural integrity or structural barriers. The matrix may be polymeric, non-polymeric, hydrophobic, hydrophilic, lipophilic, amphiphilic, and the like.

The term "openings" includes both through openings and recesses.

The term "pharmaceutically acceptable" refers to the characteristic of being non-toxic to a host or patient and suitable for maintaining the stability of a beneficial agent and allowing the delivery of the beneficial agent to target cells or tissue.

The various embodiments of the invention described herein provide different beneficial agents in different openings in the expandable device or beneficial agent in some openings and not in others. The particular structure of the expandable medical device may be varied without departing from the invention. Since each opening is filled independently, individual chemical compositions and pharmacokinetic properties can be imparted to the beneficial agent in each opening.

One example of the use of different beneficial agents in different openings in an expandable medical device or beneficial agents in some openings and not in others, is in addressing edge effect restenosis. As discussed above, current generation coated stents have a significant problem with edge effect restenosis or restenosis occurring just beyond the edges of the stent and progressing around the stent and into the interior luminal space.

The causes of edge effect restenosis in first generation drug delivery stents are currently not well understood. It may be that the region of tissue injury due to angioplasty and/or stent implantation extends beyond the diffusion range of current generation beneficial agents such as paclitaxel and Rapamycin, which tend to partition strongly in tissue. A similar phenomenon has been observed in radiation therapies in which low doses of radiation at the edges of stent have proven stimulatory in the presence of an injury. In this case, radiating over a longer length until uninjured tissue is irradiated solved the problem. In the case of drug delivery stents, placing higher doses or higher concentrations of beneficial agents along the stent edges, placing different agents at the stent edges which diffuse more readily through the tissue, or placing different beneficial agents or combinations of beneficial agents at the edges of the device would help to remedy the edge effect restenosis problem.

FIG. 1 illustrates an expandable medical device 10 with "hot ends" or beneficial agent provided in the openings 30a at the ends of the device in order to treat and reduce edge effect restenosis. The remaining openings 30b in the central portion of the device may be empty (as shown) or may contain a lower concentration of beneficial agent.

Other mechanisms of edge effect restenosis may involve cytotoxicity of particular drugs or combinations of drugs. Such mechanisms could include a physical or mechanical contraction of tissue similar to that seen in epidermal scar tissue formation, and the stent might prevent the contractile response within its own boundaries, but not beyond its edges. Further, the mechanism of this latter form of restenosis may be related to sequelae of sustained or local drug delivery to the arterial wall that is manifest even after the drug itself is no longer present in the wall. That is, the restenosis may be a response to a form of noxious injury related to the drug and/or the drug carrier. In this situation, it might be beneficial to exclude certain agents from the edges of the device.

FIG. 2 illustrates an alternative embodiment of an expandable medical device 200 having a plurality of openings 230 in which the openings 230b in a central portion of the device are filled with a beneficial agent and the openings 230a at the edges of the device remain empty. The device of FIG. 2 is referred to as having "cool ends".

In addition to use in reducing edge effect restenosis, the expandable medical device 200 of FIG. 2 may be used in conjunction with the expandable medical device 10 of FIG. 1 or another drug delivery stent when an initial stenting procedure has to be supplemented with an additional stent. For example, in some cases the device 10 of FIG. 1 with "hot ends" or a device with uniform distribution of drug may be implanted improperly. If the physician determines that the device does not cover a sufficient portion of the lumen a supplemental device may be added at one end of the existing device and slightly overlapping the existing device. When the supplemental device is implanted, the device 200 of FIG. 2 is used so that the "cool ends" of the medical device 200 prevent double-dosing of the beneficial agent at the overlapping portions of the devices 10, 200.

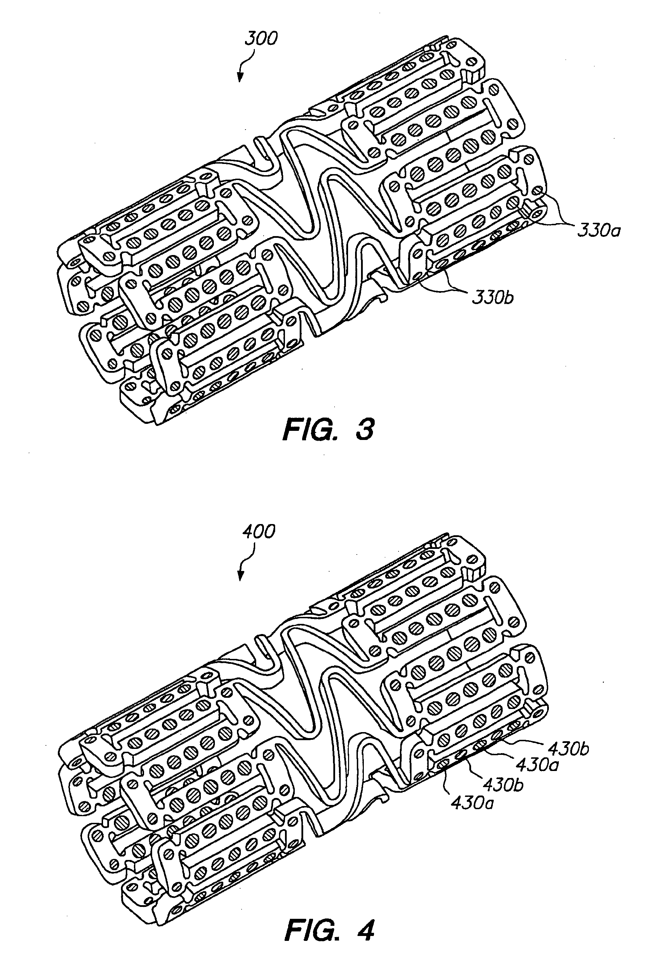

FIG. 3 illustrates a further alternative embodiment of the invention in which different beneficial agents are positioned in different holes of an expandable medical device 300. A first beneficial agent is provided in holes 330a at the ends of the device and a second beneficial agent is provided in holes 330b at a central portion of the device. The beneficial agent may contain different drugs, the same drugs in different concentrations, or different variations of the same drug. The embodiment of FIG. 3 may be used to provide an expandable medical device 300 with either "hot ends" or "cool ends."

Preferably, each end portion of the device 300 which includes the holes 330a containing the first beneficial agent extends at least one hole and up to about 15 holes from the edge. This distance corresponds to about 0.005 to about 0.1 inches from the edge of an unexpanded device. The distance from the edge of the device 300 which includes the first beneficial agent is preferably about one section, where a section is defined between the bridging elements.

Different beneficial agents containing different drugs may be disposed in different openings in the stent. This allows the delivery of two or more beneficial agents from a single stent in any desired delivery pattern. Alternatively, different beneficial agents containing the same drug in different concentrations may be disposed in different openings. This allows the drug to be uniformly distributed to the tissue with a non-uniform device structure.

The two or more different beneficial agents provided in the devices described herein may contain (1) different drugs; (2) different concentrations of the same drug; (3) the same drug with different release kinetics, i.e., different matrix erosion rates; or (4) different forms of the same drug. Examples of different beneficial agents formulated containing the same drug with different release kinetics may use different carriers to achieve the elution profiles of different shapes. Some examples of different forms of the same drug include forms of a drug having varying hydrophilicity or lipophilicity.

In one example of the device 300 of FIG. 3, the holes 330a at the ends of the device are loaded with a first beneficial agent comprising a drug with a high lipophilicity while holes 330b at a central portion of the device are loaded with a second beneficial agent comprising the drug with a lower lipophilicity. The first high lipophilicity beneficial agent at the "hot ends" will diffuse more readily into the surrounding tissue reducing the edge effect restenosis.

The device 300 may have an abrupt transition line at which the beneficial agent changes from a first agent to a second agent. For example, all openings within 0.05 inches of the end of the device may contain the first agent while the remaining openings contain the second agent. Alternatively, the device may have a gradual transition between the first agent and the second agent. For example, a concentration of the drug in the openings can progressively increase (or decrease) toward the ends of the device. In another example, an amount of a first drug in the openings increases while an amount of a second drug in the openings decreases moving toward the ends of the device.

FIG. 4 illustrates a further alternative embodiment of an expandable medical device 400 in which different beneficial agents are positioned in different openings 430a, 430b in the device in an alternating or interspersed manner. In this manner, multiple beneficial agents can be delivered to tissue over the entire area or a portion of the area supported by the device. This embodiment will be useful for delivery of multiple beneficial agents where combination of the multiple agents into a single composition for loading in the device is not possible due to interactions or stability problems between the beneficial agents.

In addition to the use of different beneficial agents in different openings to achieve different drug concentrations at different defined areas of tissue, the loading of different beneficial agents in different openings may be used to provide a more even spatial distribution of the beneficial agent delivered in instances where the expandable medical device has a non-uniform distribution of openings in the expanded configuration.

For example, in many of the known expandable devices and for the device illustrated in FIG. 5 the coverage of the device 500 is greater at the cylindrical tube portions 512 of the device than at the bridging elements 514. Coverage is defmed as the ratio of the device surface area to the area of the lumen in which the device is deployed. When a device with varying coverage is used to deliver a beneficial agent contained in openings in the device, the beneficial agent concentration delivered to the tissue adjacent the cylindrical tube portions 512 is greater that the beneficial agent delivered to the tissue adjacent the bridging elements 514. In order to address this longitudinal variation in device structure and other variations in device coverage which lead to uneven beneficial agent delivery concentrations, the concentration of the beneficial agent may be varied in the openings at portions of the device to achieve a more even distribution of the beneficial agent throughout the tissue. In the case of the embodiment of FIG. 5, the openings 530a in the tube portions 512 include a beneficial agent with a lower drug concentration than the openings 530b in the bridging elements 514. The uniformity of agent delivery may be achieved in a variety of manners including varying the drug concentration, the opening diameter or shape, the amount of agent in the opening (i.e., the percentage of the opening filed), the matrix material, or the form of the drug.

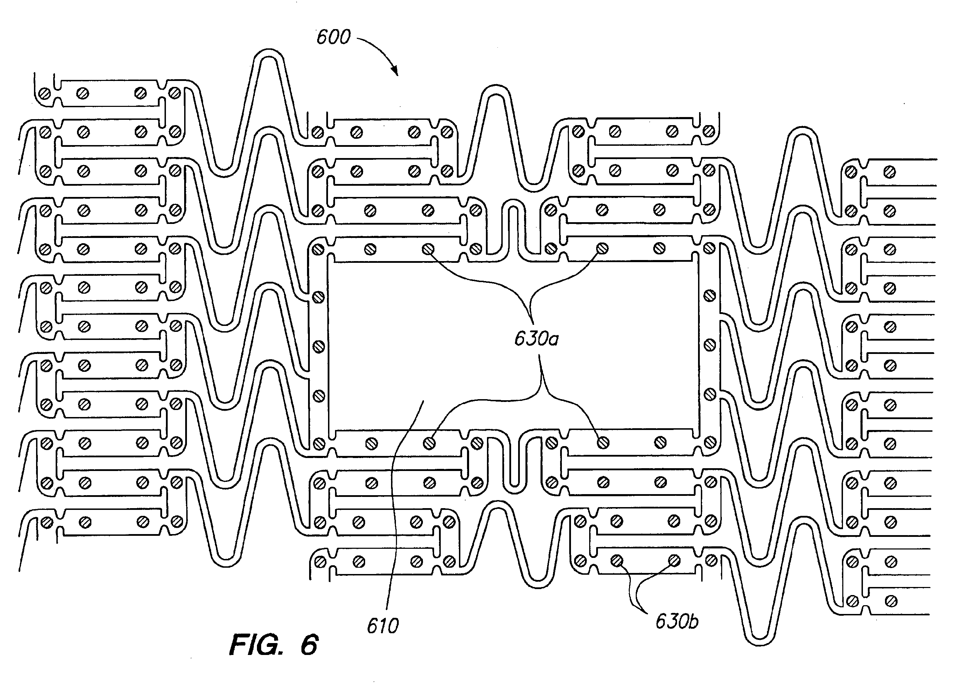

Another example of an application for the use of different beneficial agents in different openings is in an expandable medical device 600, as shown in FIG. 6, configured for use at a bifurcation in a vessel. Bifurcation devices include a side hole 610 which is positioned to allow blood flow through a side branch of a vessel. One example of a bifurcation device is described in U.S. Patent No. 6,293,967 which is incorporated herein by reference in its entirety. The bifurcation device 600 includes the side hole feature 610 interrupting the regular pattern of beams which form a remainder of the device. Since an area around a bifurcation is a particularly problematic area for restenosis, a concentration of an antiproliferative drug may be increased in openings 830a at an area surrounding the side hole 610 of the device 600 to deliver increased concentrations of the drug where needed. The remaining openings 630b in an area away from the side opening contain a beneficial agent with a lower concentration of the antiproliferative. The increased antiproliferative delivered to the region surrounding the bifurcation hole may be provided by a different beneficial agent containing a different drug or a different beneficial agent containing a higher concentration of the same drug.

In addition to the delivery of different beneficial agents to the mural side of the expandable medical device for treatment of the vessel wall, beneficial agents may be delivered to the lumenal side of the expandable medical device. Drugs which are delivered into the blood stream from the lumenal side of the device can be located at a proximal end of the device or a distal end of the device.

The methods for loading different beneficial agents into different openings in an expandable medical device may include known techniques such as dipping and coating and also known piezoelectric micro-jetting techniques. Microinjection devices may be computer controlled to deliver precise amounts of two or more liquid beneficial agents to precise locations on the expandable medical device in a known manner. For example, a dual agent jetting device may deliver two agents simultaneously or sequentially into the openings. When the beneficial agents are loaded into through openings in the expandable medical device, a lumenal side of the through openings may be blocked during loading by a resilient mandrel allowing the beneficial agents to be delivered in liquid form, such as with a solvent. The beneficial agents may also be loaded by manual injection devices.

While the invention has been described in detail with reference to the preferred embodiments thereof, it will be apparent to one skilled in the art that various changes and modifications can be made and equivalents employed, without departing from the present invention.

In summary, the subject matter described in the following paragraphs, which are numbered to allow easy reference, is part of the disclosure of the present application, each of which can be claimed in the present application, and in one or more future divisional applications there from:
(1) An expandable medical device for delivery of a beneficial agent, the device comprising: a substantially cylindrical device which is expandable from a cylinder having a first diameter to a cylinder having a second diameter; a first plurality of openings formed in the substantially cylindrical device containing a first beneficial agent for delivery to tissue, wherein the first openings are positioned on first and second ends of the cylindrical device; and a second plurality of openings formed in the substantially cylindrical device containing a second beneficial agent for delivery to tissue, wherein the second openings are positioned on a central portion of the cylindrical device between the first and second ends, and wherein the second beneficial agent is different than the first beneficial agent.
(2) The device of paragraph (1) above, wherein the second beneficial agent includes a drug which is the same as a drug included in the first beneficial agent in a different concentration.
(3) The device of paragraph (1) above, wherein the second beneficial agent includes a drug which is the same as a drug included in the first beneficial agent with a different eluting profile.
(4) The device of paragraph (1) above, wherein the first and second beneficial agents comprise different drugs.
(5) The device of paragraph (1) above, wherein the first and second beneficial agents comprise different forms of the same drug.
(6) The device of paragraph (1) above, wherein the first openings contain a first beneficial agent having a higher concentration than the second beneficial agent contained in the second openings.
(7) The device of paragraph (1) above, further comprising a third beneficial agent coated on the substantially cylindrical device.
(8) The device of paragraph (1) above, wherein the first beneficial agent is paclitaxel, or an analogue or derivative thereof.
(9) The device of paragraph (1) above, wherein the first beneficial agent is rapamycin, or an analogue or derivative thereof.
(10) A tissue supporting device comprising: a tissue supporting device body configured to support a bodily lumen; a first beneficial agent contained in first openings in the tissue supporting device for delivery to tissue, wherein the first openings are positioned on first and second ends of the device body; and a second beneficial agent contained in second openings in the tissue supporting device for delivery to tissue, wherein the second openings are positioned on a central portion of the device body between the first and second ends.
(11) The device of paragraph (10) above, wherein the second beneficial agent includes a drug which is the same as a drug included in the first beneficial agent in a different concentration.
(12) The device of paragraph (11) above, wherein the first beneficial agent is paclitaxel, or an analogue or derivative thereof.
(13) The device of paragraph (11) above, wherein the first beneficial agent is rapamycin, or an analogue or derivative thereof.
(14) The device of paragraph (10) above, wherein the second beneficial agent includes a drug which is the same as a drug included in the first beneficial agent with a different eluting profile.
(15) The device of paragraph (10) above, wherein the first and second beneficial agents comprise different drugs.
(16) The device of paragraph (10) above, wherein the first and second beneficial agents comprise different forms of the same drug.
(17) An expandable medical device for delivery of a beneficial agent, the device comprising: a device body which is expandable from an initial configuration to an expanded configuration; a side hole in the device body configured to accommodate a bifurcation in a lumen; a first plurality of openings formed in the device body containing a first beneficial agent for delivery to tissue at the expanded configuration, wherein the first openings are formed in an area surrounding the side hole; and a second plurality of openings formed in the body device in an area away from the side hole.
(18) The device of paragraph (17) above, wherein the device body includes a plurality of interconnected struts and the first and second openings are formed in the struts.
(19) The device of paragraph (17) above, wherein first beneficial agent includes a higher concentration of an anti-restenosis agent than the second beneficial agent.
(20) A method of reducing restenosis in a body passageway, the method comprising: positioning a tissue supporting device in a body passageway to support the tissue, the tissue supporting device containing a first and a second beneficial agent in openings in the device; and delivering the first beneficial agent to tissue at locations adjacent ends of the tissue supporting device and the second beneficial agent to tissue between the ends of the device to reduce restenosis.
(21) The method of paragraph (20) above, wherein the two different beneficial agents comprise the same drug in different concentrations.
(22) The method of paragraph (20) above, wherein the second beneficial agent includes a drug which is the same as a drug included in the first beneficial agent with a different eluting profile.
(23) The method of paragraph (20) above, wherein the two different beneficial agents comprise different drugs.
(24) The method of paragraph (20) above, wherein the two different beneficial agents comprise different forms of the same drug.
(25) An expandable medical device for delivery of a beneficial agent, the device comprising: a substantially cylindrical device which is expandable from a cylinder having a first diameter to a cylinder having a second diameter; a first plurality of openings formed in the substantially cylindrical device containing the beneficial agent in a first concentration; and a second plurality of openings formed in the substantially cylindrical device containing the beneficial agent in a second concentration, wherein the first and second openings are arranged to deliver a uniform distribution of a drug to the tissue of a body passageway.
(26) The device of paragraph (25) above, wherein the substantially cylindrical device includes a plurality of interconnected struts and bridging elements, the first openings are formed in the struts, and the second openings are formed in the bridging elements.
(27) The device of paragraph (25) above, wherein a volume of the first openings per unit of surface area of the expanded device is greater than a volume of the second openings per unit of surface area and the first concentration is less than the second concentration to achieve the uniform distribution of the drug.
(28) The device of paragraph (25) above, wherein the first and second openings are different sizes.
(29) A method for loading a stent with a beneficial agent, the method comprising: providing a stent with a plurality of openings; and dispensing a beneficial agent through a piezo-electric micro-jet into the plurality of openings.
(30) The method of paragraph (29) above, wherein the step of dispensing includes dispensing a first beneficial agent into a first set of the plurality of openings and dispensing a second beneficial agent into a second set of the plurality of openings

## Claims

1. A tissue supporting device comprising:
a generally cylindrical tissue supporting device body (300;400;500;600) configured to support a bodily lumen;
a plurality of first through openings (330a;430a; 530a;630a) in the tissue supporting device body (300; 400;500;600), wherein the first openings (330a;430a; 530a;630a) contain a first beneficial agent; and
a plurality of second through openings (330b;430b;530b; 630b) in the tissue supporting device body (300;400; 500;600), wherein the second openings (330b;430b;530b; 630b) contain a second beneficial agent,
**characterized in that**
the second beneficial agent is different from the first beneficial agent.

2. The tissue supporting device of claim 1, being an expandable medical device for delivery of a beneficial agent, the substantially cylindrical device body (300) being expandable from a cylinder having a first diameter to a cylinder having a second diameter;
wherein the plurality of first through openings (330a) are positioned on first and second ends of the cylindrical device body (300); and wherein the plurality of second through openings (330b) are positioned on a central portion of the cylindrical device body (300) between the first and second ends.

3. The device of Claim 2, wherein the first beneficial agent is paclitaxel or rapamycin, or an analogue or derivative of either.

4. The device of Claim 2, wherein the substantially cylindrical device (300) is a stent.

5. The tissue supporting device of claim 1, being an expandable medical device for delivery of a beneficial agent, the substantially cylindrical device body (500) being expandable from a cylinder having a first diameter to a cylinder having a second diameter;
wherein the plurality of first through openings (530a) contain the beneficial agent in a first concentration; and wherein
the plurality of second through openings (530b) contain the beneficial agent in a second concentration, wherein the first and second openings (530a,530b) are arranged to deliver a uniform distribution of a drug to the tissue of a body passageway.

6. The device of Claim 5, wherein the substantially cylindrical device (500) includes a plurality of interconnected struts and bridging elements (514), the first through openings (530a) are formed in the struts, and the second through openings (530b) are formed in the bridging elements (514).

7. The device of Claim 5, wherein a volume of the first openings (530a) per unit of surface area of the expanded device (500) is greater than a volume of second openings (530b) per unit of surface area and the first concentration is less than the second concentration to achieve a uniform distribution of the drug.

8. The device of Claim 1 or 2, wherein the second beneficial agent includes a drug which is the same as a drug included in the first beneficial agent in a different concentration, or with a different eluting profile.

9. The device of Claim 1 or 2, wherein the first and second beneficial agents comprise different drugs, or different forms of the same drug.

10. The device of Claim 1 or 2, wherein the first through openings (330a;430a;530a;630a) contain a first beneficial agent having a higher concentration than the second beneficial agent contained in the second through openings (330b;430b;530b;630b).

11. The device of Claim 1 or 2, further comprising a third beneficial agent coated on the substantially cylindrical device (300;400;500;600).

12. The device of Claim 1, wherein the plurality of first and second openings (530a,530b) have different shapes.

13. The device of Claim 1 or 5, wherein the plurality of first and second through openings (530a,530b) have different sizes.

14. The device of Claim 1, wherein the first and second through openings (430a,430b;530a,530b) are alternated or interspersed to deliver the first and second beneficial agents to an entire area supported by the tissue supporting device (400;500).

15. A method of manufacturing the tissue supporting device of one of the preceding claims, wherein the plurality of first and second through openings (330a,330b; 430a, 430b; 530a,530b; 630a,630b) are laser cut.
